# EUROPEAN PATENT SPECIFICATION

(11) **EP 2 478 355 B1**
(45) Date of publication and mention of the grant of the patent: **14.11.2018**
(21) Application number: 09849624.3
(22) Date of filing: 18.09.2009
(51) Int. Cl.: G01N 33/48, G01N 33/50

(54) **METHODS FOR DETECTING AUTODIGESTION**
VERFAHREN FÜR DEN NACHWEIS VON AUTODIGESTION
PROCÉDÉS POUR LA DÉTECTION D'AUTODIGESTION

(43) Date of publication of application: 25.07.2012
(73) Proprietor: The Regents of the University of California, Oakland, CA 94607 (US)
(72) Inventor: SCHMID-SCHONBEIN, Geert W., Del Mar California 92014 (US); DELANO, Frank, San Diego California 92110 (US)
(74) Representative: Care, Alison
(86) International application number: PCT/US2009/057521
(87) International publication number: WO 2011/034539

(56) References cited:
- WO-A1-92/01935
- WO-A2-2009/132149
- US-A1- 2006 177 813
- US-B2- 7 276 473
- PENN ALEXANDER H ET AL: "Pancreatic enzymes generate cytotoxic mediators in the intestine.", SHOCK (AUGUSTA, GA.) MAR 2007, vol. 27, no. 3, March 2007 (2007-03), pages 296-304, XP9165734, ISSN: 1073-2322
- PENN ALEXANDER H ET AL: "The intestine as source of cytotoxic mediators in shock: free fatty acids and degradation of lipid-binding proteins", AMERICAN JOURNAL OF PHYSIOLOGY - HEART AND CIRCULATORY PHYSIOLOGY, vol. 294, no. 4, April 2008 (2008-04), pages H1779-H1792, XP002689336, ISSN: 0363-6135

## Description

### TECHNICAL FIELD

This document relates to materials and methods for detecting the early onset of shock and multi-organ failure by production of tissue breakdown products in biological samples.

### BACKGROUND

Physiological shock (also referred to as "circulatory shock" or simply "shock") is a life-threatening medical emergency, and is one of the most common causes of death for critically ill people. Shock can have a variety of effects, all of which are related to problems with the body's circulatory system. In shock, entry of blood into bodily tissues ("perfusion") is decreased, such that delivery of oxygen and nutrients to tissues may be reduced to a point at which cellular metabolic needs are not met and tissues cannot function properly. In some cases, shock leads to hypoxemia and/or cardiac arrest.

Shock is a complex and continuous condition that can progress through four stages. During the initial stage, reduced blood flow to tissues can cause hypoxia, leading to cell membrane damage, anaerobic respiration by cells, lactic and pyruvic acid build-up, and systemic metabolic acidosis. During the compensatory stage, the body engages physiological (e.g., neural, hormonal, and biochemical) mechanisms that can reverse the condition, at least in part. If the cause of the shock is not successfully treated, it proceeds to the progressive (also known as "decompensating") stage, and the compensatory mechanisms begin to fail. Anaerobic metabolism continues and metabolic acidosis increases. The final, refractory (irreversible) stage of shock eventually may be reached, at which point vital organ failure has occurred and the shock can no longer be reversed, and death is imminent.

Penn AH, et al., disclosed in the journal SHOCK (2007 Mar; 27(3): 296-304) that pancreatic enzymes generate cytotoxic mediators in the intestine. They further disclose that the cytotoxicity of these digestive enzymes is undetectable in the presence of suitable inhibitors.

Penn AH and Schmid-Schonbein GW disclosed in the American Journal of Physiology - Heart and Circulatory Physiology (2008 Apr; 294(4): H1779-92) that the intestine can act as a source of cytotoxic mediators during shock conditions. Furthermore, they show that the cytotoxic mediators are free fatty acids, which are released because proteases break down free fatty acid binding proteins.

WO 2009/132149 discloses techniques for treatment of conditions related to shock. Particularly, this document describes the use protease inhibition in the lumen of the gut in principle and more specifically using specific commercially available protease inhibitors.

### SUMMARY

The present invention is defined by claim 1 disclosing a method for determining the likelihood that a mammal is experiencing autodigestion. The dependent claims depict preferred embodiments of the invention.

The present document is based in part on the discovery that reduced blood pressure and blood flow to the intestine during shock can provoke the body's digestive enzymes to break down intestinal tissue as if it were food, in a process termed "autodigestion." In addition to the hemodynamic components mentioned above, shock may be associated with pancreatic enzymes *(see*, e.g., DeLano et al. (2007) FASEB J 21 :lb421; and Schmid-Schönbein (2008) Mal. Cell Biomechanic. 5:83-95). These powerful digestive enzymes are an integral part of human digestion, and normally are kept in the intestinal lumen by the mucosal epithelial barrier. When the mucosal barrier becomes compromised (e.g., during the initial stage of shock), digestive enzymes can be transported from the lumen into the intestinal wall, where they can begin the process of autodigestion. In the process, several classes of mediators with inflammatory activity can be generated that, upon entry into the central circulation, can lead to the hallmarks of inflammation and eventually cause multi-organ failure. Thus, shock also includes an inflammatory component, although the degree of the inflammatory response during shock varies widely.

Current methods to detect inflammatory activity in shock are based on symptomatic measurements (e.g., organ dysfunction, perfusion failure, skin color changes, and stimulus reaction) or biochemical analysis (e.g., cytokine levels in plasma). These are late stage detection systems, which are unable to assist in early intervention against the destructive actions of degrading enzymes in shock and onset of autodigestion. The methods provided herein can be used to detect, in a non-invasive manner, the early onset of autodigestion. These methods can be used, for example, in emergency rooms, for military triage purposes, and for other point-of-care scenarios.

This document features a method for determining the likelihood that a mammal is experiencing autodigestion. The method can include: a) providing a biological sample from the mammal; b) analyzing the biological sample for the presence of one or more tissue breakdown products; and c) classifying the mammal as likely experiencing autodigestion if the one or more tissue breakdown products are present in the biological sample at a level higher than a control level of the one or more tissue breakdown products, or classifying the mammal as not likely experiencing autodigestion if the one or more tissue breakdown products are not present in the biological sample at a level higher than the control level of the one or more tissue breakdown products. The biological sample can be air expired by the mammal, air released from the digestive tract of the mammal, or air derived during skin evaporation from the mammal. The biological sample can be selected from the group consisting of plasma, urine, saliva, tears, sweat fluid, abdominal fluid, and cerebrospinal fluid. The mammal can be a human (e.g., a human diagnosed as having inflammatory bowel disease, autism, pancreatic inflammation and cancer, one or more small or large bowel malignancies, Alzheimer's disease, or another chronic degenerative disease associated with intestinal dysfunction).

The one or more tissue breakdown products can be selected from the group consisting of methional, pentanoic acid, 2-furfurylthiol, phenylacetaldehyde, 2,6-dimethyl-5-heptenal, (E)-2-octenal, 2-nonenal, citronellal, 2,3-diethyl-5-methylpyrazine, and 4-acetylmethylcyclohexene, putrescine, cadaverine, methanethiol, indole, skatole, and hydrogen sulfide. The analyzing can comprise using gas chromatography, liquid chromatography, mass spectroscopy, fluorescence detection, or polymeric detection. The analyzing can include using high sensitivity gas chromatography or a device with sensor technology specific for detection of small organic compounds. The control level can be the level of the one or more tissue breakdown products in a biological sample from a control mammal not experiencing autodigestion, or an average level of the one or more tissue breakdown products in biological samples from a population of control mammals not experiencing autodigestion. The method can further comprise, if the mammal is classified as likely experiencing autodigestion, treating the autodigestion by administering one or more digestive enzyme inhibitors.

The method can further include communicating information about the presence of the one or more tissue breakdown products to a medical professional. The method can comprise determining whether the mammal exhibits other symptoms of shack (e.g., symptoms of shock selected from the group consisting of organ dysfunction, perfusion failure, skin color changes, increased plasma cytokine levels, and decreased reaction to stimuli). The cytokine can he selected from the group consisting of tumor necrosis factor-a, interleukin-1, interleukin-6, and interleukin-8

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and from the claims.

### DETAILED DESCRIPTION

This document provides methods that can be used to detect early onset of shock and multi-organ failure by detecting or measuring tissue breakdown products in the expired air, as well as in gas from the intestinal tract and air derived during skin evaporation. The methods can be used, for example, during general surgery or after trauma, and can facilitate early triage treatment. The methods thus are useful for civilian and military patients. In addition, the methods also apply to patients with intestinal dysfunction (e.g., inflammatory bowel disease, autism, Alzheimer's disease, and other chronic degenerative diseases).

The methods provided herein are based in part on the discovery that shock can provoke the body's digestive enzymes begin the process of autodigestion in the intestine, resulting in production of small tissue breakdown products. The methods include detection of these enzymatic (e.g., proteolytic, lipolytic, or amylolytic) breakdown products in expired air or in biological fluid samples (e.g., plasma, urine, saliva, tears, sweat, abdominal fluid, or cerebrospinal fluid). Particularly when the methods utilize expired air or fluids such as tears, or sweat, for example, they can be non-invasive.

As described in the Example below, exposure of experimental animals to different forms of shock (e.g., hemorrhagic, endotoxin, or cecal ligation induced shock) resulted in the release of malodorous organic material from the intestine, but only if the animals were subject to autodigestion by pancreatic enzymes. Non-surviving animals uniformly released fetid organic material, while survivors produced dramatically lower levels. Mass spectrometry demonstrated that small molecular weight organic material that was present in control plasma was degraded in the plasma of shock-subjected animals, consistent with the generation of small organic degradation products in shock.

The small organic species that may be present in biological samples (e.g., air samples and fluid samples) of individuals experiencing shock can include, without limitation, any one or more of the following: methional, pentanoic acid, 2-furfurylthiol, phenylacetaldehyde, 2,6-dimethyl-5-heptenal, (E)-2-octenal, 2-nonenal, citronellal, 2,3-diethyl-5-methylpyrazine , and 4-acetylmethylcyclohexene, putrescine, cadaverine, methanethiol, indole, skatole, and hydrogen sulfide. Further details regarding these compounds is provided below. It is noted that other compounds also may be present and useful for the methods disclosed herein.

Methional (MW 104.17) has the formula C₄H₈OS, and also is known as 3-(methylthio)propionaldehyde or 4-thiapentanal. Methional has the following structure:

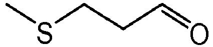

Methional is thought to be an intermediate in the reaction of methionine with sulfite during formation of methanethiol. It has a vegetable flavor, and is present in skim milk exposed to sun light. In addition, methional has been shown to be a cellular mediator of apoptosis in murine lymphoid cells (Roch et al. (1998) Cytometry 31:10-19).

Pentanoic acid (MW 102.1) has the formula C₅H₁₀O₂, and also is known as valeric acid or butane-1-carboxylic acid. Pentanoic acid has the following structure: 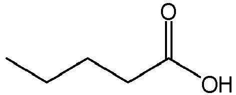 Like other low-molecular-weight carboxylic acids, pentanoic acid has a very unpleasant odor. It is found naturally in the perennial flowering plant valerian (*Valeriana officinalis*), and its primary use is in the synthesis of its esters since volatile esters of pentanoic acid tend to have pleasant odors and are used in perfumes and cosmetics.

2-furfurylthiol (MW 114.17) has the formula C₅H₆OS, and also is known as furan-2-ylmethanethiol, among others. 2-furfurylthiol is a potent odorant in heated meat, and has the following structure:

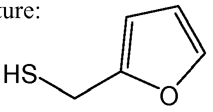

Phenylacetaldehyde (MW 120.1) has the formula C8H8O, and also is known as hyacinthin or phenylethanal. Phenylacetaldehyde has the following structure: 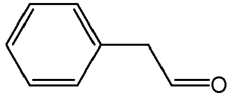 Phenylacetaldehyde is found in buckwheat, chocolate, and other foods and flowers. It also is also responsible for the antibiotic activity of maggot therapy, and can be added to cigarettes to improve their aroma. In addition, many insects use phenylacetaldehyde for communication. The aroma of pure phenylacetaldehyde has been described as honey-like, sweet, rose, green, and grassy.

2,6-dimethyl-5-heptenal (MW 140.2) has the formula C₉H₁₆O, and also is known as melonal. A food additive, 2,6-dimethyl-5-heptenal has the following structure:

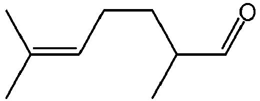

(E)-2-octenal (MW 126.20) has the formula C₈H₁₄O, and also is known as (E)-oct-2-enal. (E)-2-octenal is a lipid peroxidation product with lysine and histidine analogs, and is a mosquito attractant. This compound has the following structure:

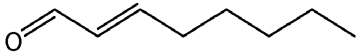

2-nonenal (MW 140.2) has the formula C₉H₁₆O, and the following structure: 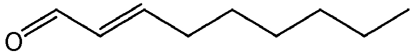 2-nonenal is a lipid peroxidation product is an unsaturated aldehyde that is a component of aged beer and buckwheat, and that may be associated with changes in human body odor that can occur during aging. It has an unpleasant greasy, grassy odor.

Citronellal (MW 154.25) has the formula C₁₀H₁₈O, and also is known as rhodinal or 3,7-dimethyloct-6-en-1-al. Citronellal has the following structure: 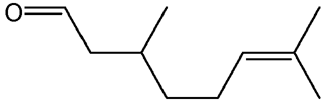 In addition, citronellal has insect repellent properties, and also has strong antifungal qualities.

2,3-diethyl-5-methylpyrazine (MW 150.2) has the formula C₉H₁₄N₂, and the following structure: 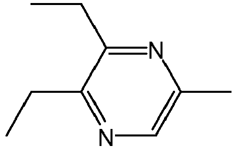 This compound is used in the food industry as a flavor ingredient, and has a potato-derived odor and flavor reminiscent of bread crust or nuts.

4-acetylmethylcyclohexene (MW 138.1) has the formula C₉H₁₄O, and the following structure:

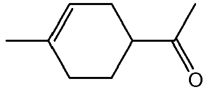

Putrescine (MW 88.2) has the formula NH₂(CH₂)₄NH₂. Putrescine is also called 1,4-diaminobutane or butanediamine, and has the following structure: 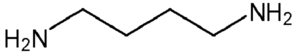 Putrescine is related to cadaverine (below), and is produced by the breakdown of amino acids in living and dead organisms. Putrescine and cadaverine are largely responsible for the foul odor of putrefying flesh, and also contribute to the odor of processes such as bad breath.

Cadaverine (MW 102.2) is a toxic, foul-smelling diamine with the formula NH₂(CH₂)₅NH₂. Cadaverine has the following structure: 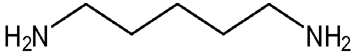 Like putrescine, cadaverine is produced by protein hydrolysis during putrefaction of animal tissue. Cadaverine also is known as 1,5-pentanediamine and pentamethylenediamine.

Methanethiol (MW 48.1) is a colorless gas that is released from decaying organic matter in marshes and has a smell like rotten cabbage. It has the formula CH₃SH, and also is known as methyl mercaptan, mercaptomethane, and thiomethyl alcohol. Methanethiol is naturally found in animal tissues such as blood and brain, in plant tissues, and in some foods (e.g., some nuts and cheese). The compound is disposed of through animal feces, and it is one of the main chemicals responsible for bad breath and the smell of flatus.

Indole (MW 117.2) has the formula C₈H₇N, and the following structure: 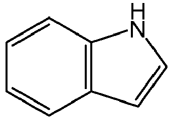 Indole also is known as 2,3-Benzopyrrole, ketole, and 1-benzazole, and is a popular component of fragrances and the precursor to many pharmaceuticals.

Skatole (MW 131.2) has the formula C₉H₉N, and the following structure: 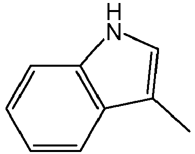 Also known as 4-methyl-2,3-benzopyrrole, skatole is a mildly toxic white crystalline organic compound that is produced from tryptophan in the mammalian digestive tract, and thus occurs naturally in feces. Skatole has a strong fecal odor, although in low concentrations it has a flowery smell and it is used as a fragrance and fixative in many perfumes. "Skatole" derives from the Greek root "skato," meaning "dung."

Hydrogen sulfide (or hydrogen sulphide; MW 34.1) has the formula H₂S. Also known as sulfur hydride, sulfurated hydrogen, and hydrosulfuric acid, among others, this toxic and flammable gas is partially responsible for the odor of rotten eggs and flatulence. Hydrogen sulfide can result from bacterial breakdown of sulfites in nonorganic matter in the absence of oxygen.

The methods provided herein can be used to determine the likelihood that a mammal (e.g., a human, a horse, a dog, a cat, a rat, or a mouse) is experiencing autodigestion. The methods can include analyzing a biological sample for the presence or levels of tissue breakdown products including, for example, hydrogen sulfide, cadeverine, putrescine, and the other compounds listed herein. Suitable biological samples include, without limitation, expired air, plasma, urine, saliva, tears, sweat fluid, abdominal fluid, and cerebrospinal fluid. Biological samples can be obtained using techniques that are standard in the art. For example, expired air samples can be obtained using nasal clips. In some embodiments (e.g., when the biological sample is expired air), samples can be analyzed as they are obtained.

The samples can be assayed for tissue breakdown products using methods that include chromatography and/or mass spectroscopy. These methods can include, for example, high sensitivity gas chromatography, high pressure liquid chromatography, liquid chromatography with mass spectrometry, fluorescence detection, polymeric detection, and/or carbon nanotube detection systems. Devices with specific sensor technology developed for detection of selected small organic compounds also can be used. These include, without limitation, z-Nose (Electronic Sensor Technology, Newbury Park, CA; online at estcal.com), or breathalyzer (Menssana Research, Fort Lee, NJ; online at menssanaresearch.com).

The methods also can include classifying a mammal as likely experiencing autodigestion if one or more tissue breakdown products are detected in the biological sample (e.g., expired air, air passed through the digestive tract, air derived during skin evaporation, or a biological fluid), or classifying the mammal as not likely experiencing autodigestion if the one or more tissue breakdown products are not detected in the biological sample. In some cases, the method can include classifying a mammal as likely experiencing autodigestion if one or more tissue breakdown products are present in the biological sample at a level higher than a control level of the one or more tissue breakdown products (e.g., the level in a biological sample from a control mammal not experiencing autodigestion, or the average level in biological samples from a population of control mammals not experiencing autodigestion), or classifying the mammal as not likely experiencing autodigestion if the one or more tissue breakdown products are not present in the biological sample at a level higher than the control level. In some embodiments, the level of one or more tissue breakdown products can be statistically significantly higher in the biological sample from the mammal than the control level. As used herein, the term "statistically significantly higher" refers to test vs. control levels with a p value less than or equal to 0.05.

In some cases, such as when a mammal is classified as likely experiencing autodigestion, the mammal can be treated by administering one or more digestive enzyme inhibitors. Any pancreatic protease inhibitor (e.g., futhane, cyclokapron, or trasylol) can be used. These can be administered into the intestinal lumen, either alone or in combination with an intravenously infused matrix metalloproteinase inhibitor.

In some embodiments, the methods provided herein can include determining whether the mammal exhibits other symptoms of shock. For example, a mammal can be examined and monitored to determine whether it is has organ dysfunction, perfusion failure, skin color changes, and/or increased levels of one or more plasma cytokines (e.g., tumor necrosis factor-α, interleukin-1 (IL-1), IL-6, and IL-8).

In addition, the methods provided herein can be used to assist medical or research professionals in determining whether or not a mammal is likely to be experiencing autodigestion. Medical professionals can be, for example, doctors, nurses, medical laboratory technologists, and pharmacists. Research professionals can be, for example, principle investigators, research technicians, postdoctoral trainees, and graduate students. A professional can be assisted by (1) detecting or measuring the level of a tissue breakdown product in a biological sample, and (2) communicating information about the detection or measured level to that professional.

After the information is reported, a medical professional can take one or more actions that can affect patient care. For example, a medical professional can record the information in a patient's medical record. In some cases, a medical professional can record a diagnosis of shock and/or autodigestion, or otherwise transform the patient's medical record, to reflect the patient's medical condition. In some cases, a medical professional can review and evaluate a patient's entire medical record, and assess multiple treatment strategies, for clinical intervention of a patient's condition.

A medical professional can initiate or modify treatment for autodigestion and/or shock after receiving information regarding tissue breakdown products in a patient. For example, a medical professional can administer a digestive enzyme inhibitor to a patient having detectable or increased levels of one or more tissue breakdown products. A medical professional also can communicate information regarding the likelihood of autodigestion and shock to a patient or a patient's family. In some cases, a medical professional can provide a patient and/or a patient's family with information regarding autodigestion and shock, including treatment options and prognosis. In some cases, a medical professional can provide a copy of a patient's medical records to communicate the levels of tissue breakdown products to a specialist.

A research professional can apply information regarding a patient's detected tissue breakdown products to advance research. For example, a researcher can compile data on detected levels of tissue breakdown products with information regarding the efficacy of a drug for treatment of autodigestion to identify an effective treatment.

Any appropriate method can be used to communicate information to another person (e.g., a professional). For example, information can be given directly or indirectly to a professional. For example, a laboratory technician can input information regarding the presence or levels of tissue breakdown product into a computer-based record. In some cases, information can be communicated by making an physical alteration to medical or research records. For example, a medical professional can make a permanent notation or flag a medical record for communicating a diagnosis to other medical professionals reviewing the record. In addition, any type of communication can be used to communicate the information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

This document also provides a diagnostic sensor system that can be used for point of care detection in acute shock patients. The detection of tissue breakdown products may also be of diagnostic value in a wide variety of diseases and in aging, including detection of tissue breakdown in autism of children, during pregnancy, and in adults with intestinal dysfunction (e.g., due to inflammatory bowel disease, pancreatic inflammation and cancer, malignancies in the small and large bowel, Alzheimer's disease, and other chronic degenerative diseases, such as rheumatoid arthritis). A diagnostic system can be designed to detect degradation products in fresh expired or evaporated air, or in fresh vapor released form the bowel with a direct sensor at point of care. In other applications, expired air and vapor can be collected in sealed bags to be analyzed within a period of hours in a laboratory. Control samples of ambient air, air from control individuals without evidence of disease, and/or vapor samples with known concentrations of selected organic degradation products can be collected in sealed collection bags. Control samples of ambient air, air from control individuals without evidence of disease, and vapor samples with known concentrations of selected organic degradation products can be collected at the same time to facilitate measurement calibration.

The invention will be further described in the following example, which does not limit the scope of the invention described in the claims.

### EXAMPLE

Experimental rats were exposed to hemorrhagic (two hour reduction of blood pressure by withdrawal of blood volume via the femoral artery), endotoxin (by administration of 5 mg/kg gram negative endotoxin), or peritonitis (induced by placement of 900 mg/kg cecal material into the peritoneal cavity) induced shock, and were observed for release of malodorous organic material from the intestine and in expired air. Fetid organic material was released from animals subject to autodigestion by pancreatic enzymes, but not from those that were not subject to autodigestion. Non-surviving animals uniformly released fetid organic material, while survivors produced dramatically lower levels. For example, animals with blockage of digestive enzymes in the lumen of the intestine (by infusion of pancreatic serine protease inhibitors, such as cyclokapron) in hemorrhagic shock had lower levels of substances such as putrescine, cadaverine, methional, and transthyretin.

Plasma samples were collected from the experimental rats before and after hemorrhagic shock and subjected to mass spectrometry, which demonstrated that small molecular weight organic material that was present in control plasma was degraded in the plasma of shock-subjected animals, consistent with the generation of smaller organic degradation products in shock (including degraded apolipoprotein, transthyretin, paralbumin, hemoglobin, complement, carbonic anhydrase, alpha 1 macroglobulin, and serine protease inhibitors).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claim

## Claims

1. A method for determining the likelihood that a mammal is experiencing autodigestion, comprising:
a) analyzing a provided biological sample from the mammal for the presence of one or more tissue breakdown products; and
b) classifying the mammal as likely experiencing autodigestion if the one or more tissue breakdown products are present in the biological sample at a level higher than a control level of the one or more tissue breakdown products, or classifying the mammal as not likely experiencing autodigestion if the one or more tissue breakdown products are not present in the biological sample at a level higher than the control level of the one or more tissue breakdown products,
wherein the biological samples is air expired by the mammal, plasma, urine, saliva, tears, or sweat fluid; and
wherein the one or more tissue breakdown products are selected from the group consisting of methional, pentanoic acid, 2-furfurylthiol, phenylacetaldehyde, 2,6-dimethyl-5-heptenal, (E)-2-octenal, 2-nonenal, citronellal, 2,3-diethyl-5-methylpyrazine, and 4- acetylmethylcyclohexene, putrescine, cadaverine, methanethiol, indole, skatole, and hydrogen sulfide.

2. The method of claim 1, wherein the biological sample is air expired by the mammal.

3. The method of claim 1, wherein the mammal is a human.

4. The method of claim 3, wherein the human is diagnosed as having shock.

5. The method of claim 1, wherein the analyzing comprises using gas chromatography, liquid chromatography, mass spectroscopy, fluorescence detection, or polymeric detection.

6. The method of claim 5, wherein the analyzing comprises using high sensitivity gas chromatography or a device with sensor technology specific for detection of small organic compounds.

7. The method of claim 1, wherein the control level is the level of the one or more tissue breakdown products in a biological sample from a control mammal not experiencing autodigestion.

8. The method of claim 1, wherein the control level is an average level of the one or more tissue breakdown products in biological samples from a population of control mammals not experiencing autodigestion.

9. The method of claim 1, further comprising communicating information about the presence of the one or more tissue breakdown products to a medical professional.

10. The method of claim 4, further comprising determining whether the mammal exhibits other symptoms of shock.

11. The method of claim 10, wherein the symptoms of shock are selected from the group consisting of organ dysfunction, perfusion failure, skin color changes, increased plasma cytokine levels, and decreased reaction to stimuli.

12. The method of claim 11, wherein the cytokine is selected from the group consisting of tumor necrosis factor-a, interleukin-1, interleukin-6, and interleukin-8.

## Patentansprüche

1. Verfahren zum Bestimmen der Wahrscheinlichkeit, dass ein Säugetier eine Autodigestion erfährt, umfassend:
a) Analysieren einer bereitgestellten biologischen Probe aus dem Säugetier auf das Vorhandensein eines oder mehrerer Gewebeabbauprodukte; und
b) Klassifizieren des Säugetiers als wahrscheinlich eine Autodigestion erfahrend, wenn das eine oder die mehreren Gewebeabbauprodukte in der biologischen Probe bei einem höheren Niveau als ein Kontrollniveau des einen oder der mehreren Gewebeabbauprodukte vorhanden sind, oder Klassifizieren des Säugetiers als wahrscheinlich keine Autodigestion erfahrend, wenn das eine oder die mehreren Gewebeabbauprodukte in der biologischen Probe nicht bei einem Niveau vorhanden sind, das höher ist als das Kontrollniveau des einen oder der mehreren Gewebeabbauprodukte,
wobei es sich bei den biologischen Proben um Luft, die von dem Säugetier ausgeatmet wird, Plasma, Urin, Speichel, Tränen oder Schweißflüssigkeit handelt; und
wobei das eine oder die mehreren Gewebeabbauprodukte ausgewählt sind aus der Gruppe bestehend aus Methional, Pentansäure, 2-Furfurylthiol, Phenylacetaldehyd, 2,6-Dimethyl-5-heptenal, (E)-2-Octenal, 2-Nonenal, Citronellal, 2,3-Diethyl-5-methylpyrazin und 4-Acetylmethylcyclohexen, Putrescin, Cadaverin, Methanthiol, Indol, Skatol und Schwefelwasserstoff.

2. Verfahren von Anspruch 1, wobei es sich bei der biologischen Probe um Luft handelt, die von dem Säugetier ausgeatmet wird.

3. Verfahren von Anspruch 1, wobei das Säugetier ein Mensch ist.

4. Verfahren von Anspruch 3, wobei bei dem Menschen das Vorliegen eines Schocks diagnostiziert wird.

5. Verfahren von Anspruch 1, wobei das Analysieren die Verwendung von Gaschromatographie, Flüssigkeitschromatographie, Massenspektroskopie, Fluoreszenzdetektion oder polymerer Detektion umfasst.

6. Verfahren von Anspruch 5, wobei das Analysieren das Verwenden von hochempfindlicher Gaschromatographie oder einer Vorrichtung mit Sensortechnologie, die für den Nachweis von kleinen organischen Verbindungen spezifisch ist, umfasst.

7. Verfahren von Anspruch 1, wobei das Kontrollniveau das Niveau des einen oder der mehreren Gewebeabbauprodukte in einer biologischen Probe aus einem Kontrollsäugetier ist, das keine Autodigestion erfährt.

8. Verfahren von Anspruch 1, wobei das Kontrollniveau ein durchschnittliches Niveau des einen oder der mehreren Gewebeabbauprodukte in biologischen Proben aus einer Population von Kontrollsäugetieren ist, die keine Autodigestion erfahren.

9. Verfahren von Anspruch 1, ferner umfassend das Übermitteln von Informationen über das Vorhandensein des einen oder der mehreren Gewebeabbauprodukte an einen medizinischen Fachmann.

10. Verfahren von Anspruch 4, ferner umfassend das Bestimmen, ob das Säugetier andere Symptome von Schock zeigt.

11. Verfahren von Anspruch 10, wobei die Symptome von Schock ausgewählt sind aus der Gruppe bestehend aus Organdysfunktion, Perfusionsversagen, Hautfarbveränderungen, erhöhten Plasmazytokinniveaus und verminderter Reaktion auf Stimuli.

12. Verfahren von Anspruch 11, wobei das Zytokin aus der Gruppe bestehend aus Tumornekrosefaktor-a, Interleukin-1, Interleukin-6 und Interleukin-8 ausgewählt ist.

## Revendications

1. Méthode de détermination de la probabilité qu'un mammifère souffre d'une autodigestion, comprenant :
a) l'analyse d'un échantillon biologique fourni et issu du mammifère pour détecter la présence d'un ou plusieurs produits de dégradation tissulaire ; et
b) la classification du mammifère comme souffrant probablement d'une autodigestion, si les un ou plusieurs produits de dégradation tissulaire sont présents dans l'échantillon biologique selon un taux supérieur à un taux témoin des un ou plusieurs produits de dégradation tissulaire, ou la classification du mammifère comme ne souffrant probablement pas d'une autodigestion, si les un ou plusieurs produits de dégradation tissulaire ne sont pas présents dans l'échantillon biologique selon un taux supérieur au taux témoin des un ou plusieurs produits de dégradation tissulaire ;
où l'échantillon biologique est constitué par l'air expiré par le mammifère, le plasma, l'urine, la salive, les larmes, ou la sueur ; et
où les un ou plusieurs produits de dégradation tissulaire sont choisis dans le groupe constitué par le méthional, l'acide pentanoïque, le 2-furfurylthiol, le phénylacétaldéhyde, le 2,6-diméthyl-5-heptanal, le (E)-2-octénal, le 2-nonénal, le citronellal, la 2,3-diéthyl-5-méthylpyrazine, et le 4-acétylméthylcyclohexène, la putrescine, la cadavérine, le méthanethiol, l'indole, le scatole, et le sulfure d'hydrogène.

2. Méthode selon la revendication 1, dans laquelle l'échantillon biologique est constitué par l'air expiré par le mammifère.

3. Méthode selon la revendication 1, dans laquelle le mammifère est un être humain.

4. Méthode selon la revendication 3, dans laquelle l'être humain est diagnostiqué comme souffrant de choc.

5. Méthode selon la revendication 1, dans laquelle l'analyse comprend l'utilisation de chromatographie gazeuse, de chromatographie liquide, de spectroscopie de masse, de détection par fluorescence, ou de détection polymère.

6. Méthode selon la revendication 5, dans laquelle l'analyse comprend l'utilisation de chromatographie gazeuse à haute sensibilité ou d'un dispositif ayant une technologie de capteur spécifique pour la détection de composés organiques de petite taille.

7. Méthode selon la revendication 1, dans laquelle le taux témoin est le taux des un ou plusieurs produits de dégradation tissulaire dans un échantillon biologique issu d'un mammifère témoin ne souffrant pas d'une autodigestion.

8. Méthode selon la revendication 1, dans laquelle le taux témoin est le taux moyen des un ou plusieurs produits de dégradation tissulaire dans des échantillons biologiques issus d'une population de mammifères témoins ne souffrant pas d'une autodigestion.

9. Méthode selon la revendication 1, comprenant en outre la communication d'informations concernant la présence des un ou plusieurs produits de dégradation tissulaire à un professionnel de santé.

10. Méthode selon la revendication 4, comprenant en outre la détermination du fait que le mammifère présente ou non d'autres symptômes de choc.

11. Méthode selon la revendication 10, dans laquelle les symptômes de choc sont choisis dans le groupe constitué par le dysfonctionnement d'organes, l'échec de perfusion, les changements de couleur de la peau, les taux accrus en cytokines plasmatiques, et une réaction réduite vis-à-vis de stimuli.

12. Méthode selon la revendication 11, dans laquelle les cytokines sont choisies dans le groupe constitué par le facteur de nécrose tumorale a, l'interleukine 1, l'interleukine 6, et l'interleukine 8.
